Europäisches Patentamt

**European Patent Office**

Office européen des brevets

(11) Publication number: **0 223 345 B1**

# EUROPEAN PATENT SPECIFICATION

(45) Date of publication of the patent specification: 26.09.90

(51) Int. Cl.⁵: **C07C 2/00, B01J 29/28**

(21) Application number: **86306785.6**

(22) Date of filing: **02.09.86**

(54) Process for the conversion of light olefins into heavier hydrocarbons.

(30) Priority: **17.09.85 US 777049**

(43) Date of publication of application: **27.05.87 Bulletin 87/22**

(45) Publication of the grant of the patent: **26.09.90 Bulletin 90/39**

(84) Designated Contracting States: **BE DE FR GB IT NL**

(56) References cited:
**EP-A- 0 050 021
EP-A- 0 150 105
WO-A-84/03879
FR-A- 2 374 283**

The file contains technical information submitted after the application was filed and not included in this specification

(73) Proprietor: **MOBIL OIL CORPORATION, 150 East 42nd Street, New York New York 10017(US)**

(72) Inventor: **Chang, Clarence Dayton, 11 Murray Place, Princeton New Jersey 08540(US)**
Inventor: **Chu, Cynthia Ting-Wah, 283 Westcott Boulevard, Pennington New Jersey 08534(US)**

(74) Representative: **Cooper, John Anthony et al, Mobil Court 3 Clements Inn, London WC2A 2EB(GB)**

ACTORUM AG

## Description

The invention relates to converting $C_2$–$C_5$ olefins to gasoline, distillate and lube range hydrocarbons.

Zeolites, which are crystalline aluminosilicates, have long been used for cracking, isomerization, hydrocracking, and the like.

It is also known from EP-A 150 105 to convert olefins to liquid fuel over the metallosilicate zeolite theta-1, where the metal is Al, Ga, Zn, Fe, Cr and/or B.

The invention resides in the discovery that certain crystalline silicates, having a characteristic X-ray diffraction pattern of ZSM-5, convert $C_2$–$C_5$ olefins to gasoline, distillate and lube range hydrocarbons with decreased light gas production. The alpha-value of the crystalline silicate is not critical. Thus, the invention differs from U.S. 3 960 978, where $C_2$–$C_5$ oligomerization requires a zeolite with an alpha of 0.1 to 120 at WHSV from 0.1 to 25 and temperature of 260 to 482°C (500 to 900°F).

Accordingly, the present invention provides a process for converting an olefin containing two to five carbon atoms to higher molecular weight hydrocarbons comprising contacting the olefin at 177 to 316°C (350 to 600°F) with a catalyst comprising a crystalline silicate having the X-ray diffraction pattern of ZSM-5 and containing from 0 to 0.1 weight % $Al_2O_3$ and 0.1 to 15 wt.% gallium, wherein at least part of the gallium is tetrahedrally coordinated in the crystal framework of the silicate.

The method produces less light gas and more distillate and lube boiling range hydrocarbons than methods which employ [Al] ZSM-5 of comparable value which is free of gallium. The gallium is preferably framework gallium. The crystal lattice framework of zeolites can be described as a rigid three-dimensional framework of $SiO_4$ and $AlO_4$ in which the tetrahedra are cross-linked by the sharing of oxygen atoms whereby the ratio of the total aluminium and silicon atoms to oxygen atoms is one:two. Framework gallium is a substitute for the Si of $SiO_4$ or for Al of the $AlO_4$ tetrahedra.

The present invention is directed to the catalytic conversion of a $C_2$-$C_5$ olefin, mixtures thereof, and mixtures thereof with paraffins to higher molecular weight products. The feed may also contain other light hydrocarbons, e.g., $C_2$-$C_5$ aliphatics. The products include gasoline distillate and lube range hydrocarbons. The amount of light gases produced, including $C_1$-$C_5$ aliphatics, is substantially reduced, The products are characterized by a high ratio of 343°C+ (650°F+) to $C_1$-$C_5$ aliphatics, relative to prior art processes.

Reaction conditions include temperatures from 177°C to 316°C (350 to 600°F), preferably 232°C to 271°C (450 to 520°F) and most preferably temperatures high enough to achieve conversion of most of the olefins, but below 255-260°C, to minimize formation of aromatics and enhance the production of heavy hyrocarbons suitable for use as lubricant stock. Pressures may vary widely, and preferably range from 3500 to 14 000 kPa (500 to 2000 psig). The weight hourly space velocity (WHSV) may range from 0.1 to 25.

The relatively low temperatures used in the process of the present invention are important in maximizing production of lube stocks and minimizing formation of aromatics and olefins.

The catalyst is a gallium containing crystalline silicate or a gallium containing crystalline alumino silicate, a zeolite, having a characteristic X-ray diffraction pattern. The gallium content can be expressed in terms of wt% Ga, and can range from 0.1 to 15%. In a preferred embodiment the X-ray diffraction corresponds to that of ZSM-5, as disclosed in U.S. No. 3 702 886.

In general, ZSM-5 can be prepared from a solution containing water, tetrapropyl ammonium hydroxide and the elements of sodium oxide, an oxide of aluminum or gallium, an oxide of silica, and having a composition in terms of mole ratios of oxides, falling within the following ranges:

|  | Broad | Preferred | Particularly Preferred |
|---|---|---|---|
| $OH/SiO_2$ | 0.07–0.8 | 0.1–0.8 | 0.2–0.75 |
| $R_4N+(R_4N^+ +Na^+)$ | 0.2–0.95 | 0.3–0.9 | 0.4–0.9 |
| $H_2O/OH-$ | 10–300 | 10–300 | 10–300 |
| $YO_2/W_2O_3$ | 5–100+ | 10–60 | 10–40 |

wherein R is propyl, W is aluminum and Y is silicon. This mixture is maintained at reaction conditions until the crystals of the zeolite are formed. Thereafter the crystals are separated from the liquid and recovered. Typical reaction conditions consist of a temperature of 75°C to 175°C for six hours to 60 days. A preferred temperature range is 90° to 150°C, for 12 hours to 20 days. In the examples below the preparation of ZSM-5 was modified to replace aluminum with gallium. Various sources of gallium may be used, $Ga_2(SO_4)_3$ was used in the examples below. The solid product is separated from the reaction mixture by cooling it to room temperature, filtering and water washing. Any other method for preparing a gallosilicate may also be used. The gallosilicate zeolite can also be prepared by treatment of high silica zeolite with a source of Ga in aqueous solution with pH 7 at reflux or lower temperatures; the composite is then ammonium exchanged and calcined to produce a catalyst with increased Bronsted acidity due to the inserted framework elements. This alternative method produces a more crystalline gallosilicate than is possible by crystallization from gels containing hydroxygallium constituents.

The reaction mixture can be prepared with materials which supply the elements of the appropriate oxide. Such materials include an aluminosilicate, sodium aluminate, alumina, sodium silicate, silica hydrosil, silica gel, silicic acid, sodium hydroxide and tetrapropylammonium hydroxide. Each oxide component of the reaction mixture may be supplied by one or more initial reactants. The reaction mixture can be prepared batchwise or continuously. Crystal size and crystallization time will vary with the nature of the reaction mixture employed.

The as-synthesized crystalline silicates or crystalline aluminosilicates may contain cations, which are undesirable. Replacement techniques are well known in the art. Typical replacing cations include hydrogen, ammonium and one or more metal cations, or mixtures thereof. Specific replacing cations include hydrogen, zinc, nickel, platinum, palladium, rhenium, and chromium. Representative ion exchange techniques are disclosed in U.S. 3 140 249; 3 140 251 and 3 140 253.

Following cation replacement the crystalline material may be washed with water and dried at 66°C to 316°C (150°F to 600°F) and then heated in air or other gas at 260°C to 816°C (500° F to 1500°F) for 1 to 48 hours or more.

The crystalline material may then be treated with steam at 427°C to 871°C (800°F to 1600°F), preferably 538°C to 816°C (l000°F to 1500°F), if desired. The treatment may be accomplished in atmospheres consisting partially or entirely of steam. Similar treatments can be accomplished at lower temperatures at elevated pressures, for example, at 177 to 371°C (350 to 700°F) at 10 to about 200 atmospheres.

The alpha-value of the crystalline material containing framework gallium is not critical; it preferably ranges from 5 to 300.

The Alpha Value is an approximate indication of the catalytic cracking activity of the catalyst compared to a standard catalyst. It gives the relative rate of normal hexane conversion per volume of catalyst per unit time. It is based on the activity of the highly active silica alumina cracking catalyst taken as an Alpha of 1 (Rate Constant = 0.16 sec$^{-1}$). The Alpha Test is described in U.S. Patent 3 354 078 and in The Journal of Catalysis, Vol. IV. pp. 522-529 (August 1965). The silicate tested in this Alpha Test must be at least partially in the hydrogen form. Conversion to the hydrogen form may be accomplished by contact with an ammonium salt or acid solution followed by thermal treatment to eliminate ammonia and water.

To achieve high olefin conversion to 343°C (650°F) plus hydrocarbons, crystalline aluminosilicates, e.g. ZSM-5, free of gallium, must have high alpha-values. By comparison, crystalline materials containing framework gallium, of comparable alpha-value, produce less light gas and more 343°C (650°F+) hydrocarbons. Generally, the crystalline silicates containing framework gallium may contain up to 0.1 wt.% $Al_2O_3$.

Although the crystalline silicates containing framework gallium, used in the examples are used neat, without a porous matrix, the crystalline materials may be used in combination, dispersed or otherwise intimately admixed with a porous binder or matrix, so that the resulting product contains from 1% to 95% by weight, and preferrably from 20 to 80% by weight, of the gallium silicate (or gallium aluminosilicate)in the final composite.

The terms "binder" and "matrix" refer to inorganic compositions with which the silicate can be combined, disbursed or otherwise intimately admixed. Such binders impart strength and attrition resistance to the relatively severe conditions under which the silicate is used. Representative binder or matrix materials include metals and alloys thereof, sintered metals and sintered glass, asbestos, silicon carbide aggregates, pumice, firebrick, diatomaceous earths and inorganic oxides. Inorganic compositions especially those of a siliceous nature are preferred. Of these matrices, inorganic oxides such as clay, chemically treated clay, silica, alumina, and silica-alumina are particularly preferred.

The following examples show conversion of $C_2$ - $C_5$ olefins.

## EXAMPLE 1

(a) A standard procedure for producing ZSM-5 composition was followed except the source of aluminum, $Al_2(SO_4)_3$, was replaced by $Ga_2(SO_4)_3$ as a source of gallium. . The silica source was 50 grams Q brand silica in 50 grams of water. The gallium source was a solution of 1.11 grams $Ga_2(SO_4)_3$, 84.2 grams water and 5.3 grams of $H_2SO_4$; 6.16 grams of TPA Br (tetrapropylammonium bromide) were used. The Si/$Ga_2$ ratio of the reaction mixture was about 90. The reaction mixture was stirred for several days at 160°C, until product crystallized.

(b) The silica source in the 1(a) preparation was replaced by 13.9 grams of extracted $SiO_2$ in 86 grams of water. The amounts of other components remained the same. Stirring of the components was for seven days with addition of 20cc of 10N NaOH to get a pH of about 10.

(c) The 1(b) preparation was repeated, but the amount of extracted silica was doubled.

The catalyst compositions are set forth in Table 1. In the Table, Meq/g ash means the milliequivalents of ammonia per gram of catalyst, based on weight of catalyst after calcining at 1000°C. Both calculated and experimental values are reported. The experimental value was determined using the well known TPD technique, or Temperature Programmed Desorption of ammonia. The ratio of the experimental to calculated values, expressed as %, is a measure of how much gallium is in the crystal framework.

TABLE 1

| Catalyst | (a) | (b) | (c) |
|---|---|---|---|
| Elemental Analysis wt% | | | |
| $SiO_2$ | 97.7 | 96.0 | 95.1 |
| $Al_2O_3$ (ppm) | 3700 | (86) | (95) |
| Ga | 2.38 | 2.81 | 1.41 |
| Exchange site calc. Meq/g ash | 0.415 | 0.404 | 0.206 |
| TPD meq/g ash | 0.416 | 0.322 | 0.1955 |
| % Ga in framework | 100 | 80 | 95 |
| Alpha | 55 | 60 | 40 |

Each catalyst above exhibited high crystallinity ZSM-5 X-ray diffraction patterns.

(d) In this preparation a 6 g sample of HZSM-5 ($SiO_2/Al_2O_3$ = 26 000/l) was mixed with 300cc 0.2N NaOH solution containing 1.5 g of $Ga_2(SO_4)_3$. The mixture was refluxed for 2 hours and then washed, converted into the ammonium form by exchange with $NH_4NO_3$. The resultant zeolite has an exchange capacity of 0.5446 meq/g ash and n-hexane cracking activity of 803.

## EXAMPLE 2

ZSM-5 containing gallium in the framework, prepared as in preparation (c) of Example 1, converted propylene to gasoline distillate and lube range hydrocarbons. ZSM-5 containing no gallium and having an alpha value of 55, was also tested for propylene conversion. All tests were run at 230°C and pressure of (1500 psig) 10 400 kPa. The results are tabulated in Table 2:

TABLE 2

| Catalyst | [Ga]ZSM-5* (Invention) | [Al]ZSM-5 (Prior Art) |
|---|---|---|
| Alpha | 40 | 55 |
| WHSV | 0.4 | 0.5 |
| Total Hours | 21 | 22 |
| Products (wt%) | | |
| $C_1$-$C_5$ | 2.3 | 13.0 |
| $C_6$-166°C(330°F) | 9.9 | 17.4 |
| 166°C to 343°C (330-650°F) | 58.2 | 58.3 |
| 343°C+ (650°F+) | 29.6 | 11.3 |

ZSM-5 containing framework gallium produces less light gas and more 343°C+ (650°F+) hydrocarbons than ZSM-5 free of gallium with comparable alpha value.

Table 3 below presents comparisons of the 343°C (650°F) plus product from runs at 230°C reactor temperature in which catalyst compositions employing ZSM-5, free of gallium, of differing alpha values are employed:

TABLE 3

| Catalyst | [Ga]ZSM-5 | | [Al]ZSM-5 | | |
|---|---|---|---|---|---|
| $SiO_2/M_2O_3$ | | 180 | 70/1 | 40/1 | Steamed 70/1 |
| $M_2O_3$ wt% | | 1.89 | 2.37 | 4.25 | |
| Alpha | | 40 | 180 | 400 | 55 |
| Hours | 21 | 47 | 19 | 41 | 22 |
| WHSV | | 0.4 | | 0.5 | |
| $C_1-C_5$ | 2.3 | 2.2 | 7.0 | 9.7 | 13.0 |
| $C_6$-166°C(330°F) | 9.9 | 9.9 | 16.7 | 10.8 | 17.4 |
| 166°C-343°C | 58.2 | 60.2 | 52.1 | 47.7 | 58.3 |
| 343°C+(650°F+) | 29.6 | 27.7 | 24.2 | 31.6 | 11.3 |

ZSM-5, free of gallium, must have a much higher alpha value than [Ga]ZSM-5 to achieve comparable conversion to 343°C+ (650°F+) product. The [Ga]ZSM-5 makes less $C_1$-$C_5$.

The gallium is preferably framework gallium, at least 80% framework gallium giving very good results.

The relatively low temperatures used aid in producing a heavy liquid fraction of 343°C+ material (650°F+) which is a good lubricating oil base stock, one which can be characterized as containing less than 10 wt. % aromatics, preferably less than 5 wt. % aromatics.

## Claims

1. A process for converting an olefin containing two to five carbon atoms to higher molecular weight hydrocarbons comprising contacting the olefin at 177 to 316°C (350 to 600°F) with a catalyst comprising a crystalline silicate having the X-ray diffraction pattern of ZSM-5 and containing from 0 to 0.1 weight % $Al_2O_3$ and 0.1 to 15 wt. % gallium, wherein at least part of the gallium is tetrahedrally coordinated in the crystal framework of the silicate.

2. The process of Claim 1 wherein said contacting is effected at 232 to 271°C (450 to 520°F).

3. The process Claim 1 wherein said contacting is effected at a pressure of 3500 to 14 000 kPa (500 to 2000 psig).

4. The process of any preceding claim further characterized in that the weight hourly space velocity (WHSV) of olefin to catalyst is 0.1 to 25.

5. The process of any preceding claim wherein at least 80% of the gallium on the catalyst is framework gallium.

## Patentansprüche

1. Verfahren zur Umwandlung eines Olefins, das 2 bis 5 Kohlenstoffatome enthält, in Kohlenwasserstoffe mit höherem Molekulargewicht, das den Kontakt des Olefins bei 177 bis 316°C (350 bis 600°F) mit einem Katalysator umfaßt, der ein kristallines Silicat mit einem Röntgenbeugungsdiagramm von ZSM-5 umfaßt und von 0 bis 0,1 Gew.-% $Al_2O_3$ und 0,1 bis 15 Gew.-% Gallium enthält, wobei mindestens ein Teil des Galliums in dem Kristallgitter des Silicats tetraedrisch gebunden ist.

2. Verfahren nach Anspruch 1, worin der Kontakt bei 232 bis 271°C (450 bis 520°F) durchgeführt wird.

3. Verfahren nach Anspruch 1, worin der Kontakt bei einem Druck von 3500 bis 14 000 kPa (500 bis 2000 psig) durchgeführt wird.

4. Verfahren nach einem der vorstehenden Ansprüche, weiterhin dadurch gekennzeichnet, daß die stündliche Gewichts-Raum-Geschwindigkeit (WHSV) des Olefins zum Katalysator 0,1 bis 25 beträgt.

5. Verfahren nach einem der vorstehenden Ansprüche, worin mindestens 80% des Galliums auf dem Katalysator Gitter-Gallium ist.

## Revendications

1. Procédé de conversion d'oléfines contenant de 2 à 5 atomes de carbone en hydrocarbures de poids moléculaire plus élevé, comprenant la mise en contact de la charge d'oléfines à une température de 177 à 316°C (350 à 600°F) avec un catalyseur contenant un silicate cristallin ayant le spectre de diffraction aux rayons-X de la ZSM-5 et contenant de 0 à 0,1% en poids d'$Al_2O_3$, de 0,1 à 15% en poids de gallium, et dans lequel au moins une partie du gallium est coordonnée dans les tétraèdres de la structure cristalline du silicate.

2. Le procédé de la revendication 1, dans lequel ladite mise en contact est effectuée à une température de 232 à 271°C (450 à 520°F).

3. Le procédé de la revendication 1, dans lequel ladite mise en contact est effectuée sous une pression de 3 500 à 14 000 kPa (500 à 2 000 psi).

4. Le procédé selon l'une quelconque des revendications précédentes, caractérisé de plus en ce que la vitesse spatiale horaire pondérale WHSV (masse de charge oléfinique entrant dans le réacteur par masse de catalyseur et par heure) est de 0,1 à 25.

5. Le procédé selon l'une quelconque des revendications précédentes, dans lequel au moins 80% du gallium dans le catalyseur est du gallium structurel.